# EUROPÄISCHE PATENTSCHRIFT

(11) **EP 0 865 422 B1**
(45) Veröffentlichungstag und Bekanntmachung des Hinweises auf die Patenterteilung: **11.07.2001**
(21) Anmeldenummer: 96941028.1
(22) Anmeldetag: 26.11.1996
(51) Int. Cl.: C07C 233/18, C07C 233/20, A61K 31/16

(54) **VERWENDUNG VON AMINOALKOHOLDERIVATEN ALS ARZNEIMITTEL, DERIVATE SOWIE VERFAHREN ZU DEREN HERSTELLUNG**
USE OF AMINOALCOHOLDERIVATIVES AS DRUGS, DERIVATIVES AND PROCESS FOR THEIR PREPARATION
UTILISATION DE DERIVES D'AMINOALCOLS, DERIVES ET PROCEDE POUR LEUR PREPARATION

(30) Priorität: 30.11.1995 DE 19544635
(43) Veröffentlichungstag der Anmeldung: 23.09.1998
(73) Patentinhaber: Roche Diagnostics GmbH, 68298 Mannheim (DE)
(72) Erfinder: ESSWEIN, Angelika, D-64572 Büttelborn (DE); KLING, Lothar, D-68167 Mannheim (DE); LESER, Ulrike, D-80796 München (DE); FRIEBE, Walter-Gunar, D-68165 Mannheim (DE)
(86) Internationale Anmeldenummer: EP9605212
(87) Internationale Veröffentlichungsnummer: WO9719909

(56) Entgegenhaltungen:
- EP-A- 0 625 522
- WO-A-95/08529
- DE-A- 3 309 948
- US-A- 3 795 611
- US-A- 3 846 449
- JOURNAL OF LIQUID CHROMATOGRAPHY, Bd. 10, Nr. 13, 1987, Seiten 2939-2950, XP000645787 ALBERT A. BEN-BASSAT ET AL.: "Separation and Determination of Monoalkanolamides of Soybean Oil Fatty Acids by RP-HPLC of the Crude Reaction Product"
- LIPIDS, Bd. 7, Nr. 1, 1972, Seiten 56-59, XP000645675 R. C. ARORA ET AL.: "Stimulation in Vitro of Galactocerebroside Galactosidase by N-Decanoyl-2-Amino-2-Methylpropanol"
- JOURNAL OF MEDICINAL CHEMISTRY Bd. 37, Nr. 12, 10 Juni 1994, WASHINGTON US, Seiten 1889 - 1893, XP002027261 VASILIKI ABADJI ET AL.: "(R)--Methanandamide: A Chiral Novel Anandamide Possessing Higher Potency and Metabolic Stability"
- CHEMICAL AND PHARMACEUTICAL BULLETIN Bd. 40, Nr. 1, Januar 1992, TOKYO JP, Seiten 122 - 126, XP002027262 TAMEO IWASAKI ET AL.: "A Synthesis of 2-Substituted 2-Aminoethanol Derivatives Having Inhibitory Activity against Protein Kinase C"

## Beschreibung

Die vorliegende Erfindung betrifft die Verwendung von Aminoalkoholderivaten zur Herstellung von Arzneimitteln zur Behandlung von Knochenerkrankungen, neue Aminoalkoholderivate sowie Verfahren zu deren Herstellung.

Bei gesunden Personen befinden sich die Auf- und Abbauprozesse im Knochen nahezu im Gleichgewicht, d.h. die Aktivität der Osteoblasten und Osteoklasten ist ausgeglichen. Ist dieses Gleichgewicht aber zugunsten der Osteoklasten und/oder zuungunsten der Osteoblasten gestört, kommt es zu einer Reduktion der Knochenmasse und zu einer negativen Veränderung der Knochenstruktur und -funktion.

Zur Behandlung von Knochenstoffwechselstörungen werden bisher vor allem Knochenresorptionsinhibitoren wie Oestrogene, Calcitonin und Bisphosphonate eingesetzt. Die Anwendung dieser Substanzen ist jedoch limitiert und zeigt auch nicht in allen Fällen den gewünschten Effekt. Verbindungen, die stimulierend auf den Knochenaufbau wirken und dazu beitragen eine bereits verminderte Knochenmasse zu erhöhen, sind deshalb für die Behandlung von Knochenstoffwechselstörungen von außerordentlicher Bedeutung. In den europäischen Patentanmeldungen EP-A-625522 und EP-A-524023 wurden Substanzen mit einer osteoanabolen Wirkung zur Therapie der Osteoporose beschrieben.

Überraschender Weise wurde nun gefunden, daß Aminoalkoholderivate der vorliegenden Erfindung stimulierend auf den Knochenaufbau wirken und damit zur breiten Behandlung von Knochenstoffwechselstörungen geeignet sind. Sie zur breiten Behandlung von Knochenstoffwechselstörungen geeignet sind. Sie lassen sich vor allem dort gut einsetzen, wo der Knochenaufbau gestört ist, d.h. sie sind geeignet zur Behandlung von osteopenischen Erkrankungen des Skelettsystems wie z.B. Osteoporose, u.a. von Osteogenesis imperfecta, aber auch zur Unterstützung der Knochenregeneration und Osteoinduktion wie z.B. in orthopädischen und kieferheilkundlichen Indikationen, bei Frakturheilung, Osteosynthesen, Pseudoarthrosen und Einheilung von Knochenimplantaten.

Aufgrund dieser Eigenschaften finden sie aber auch Verwendung in der Prophylaxe der Osteoporose.

Durch ihre Beeinflussung des Knochenstoffwechsels bilden sie weiterhin eine Grundlage für die Behandlung der rheumatoiden Arthritis, der Osteoarthritis und der degenerativen Arthrose.

Gegenstand der vorliegenden Erfindung sind neue Verbindungen der allgemeinen Formel (Ia), 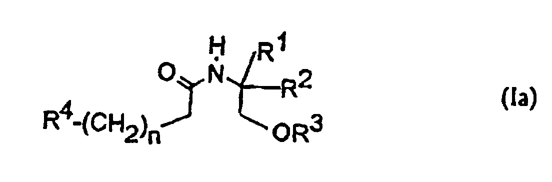 in der
- R¹ =: Wasserstoff oder Methyl,
wobei für den Fall, daß R² = Methyl, R¹ = Wasserstoff ist
- R² =: niederes geradkettiges oder verzweigtes Alkyl mit 1 bis 10 Kohlenstoffatomen
- R³ =: Wasserstoff oder niederes Alkyl mit 1 bis 6 Kohlenstoffatomen
- n =: 0-12
- R⁴ =: Alkyl, Alkenyl oder Alkinyl mit 6 bis 24 Kohlenstoffatomen,
- R⁴: Alkyl bedeutet, -(CH₂)ₙ-R⁴ keine unverzweigte Alkylkette mit 8, 10, 12, 14 oder 16 Kohlenstoffatomen sein darf,
und für den Fall, daß
- R²: Methyl, Isopropyl oder Isobutyl bedeutet, -(CH₂)ₙ-R⁴ nicht (all-*cis*-4,7,10,13)- Octadecatetraen sein darf, und keine unverzweigte Kette mit 6 Kohlenstoffatomen sein darf,
sowie deren pharmakologisch unbedenkliche Salze und optischen Isomeren.

Ferner sind Gegenstand der Erfindung Arzneimittel, die diese Verbindungen neben üblichen Träger- und Hilfsstoffen enthalten.

In EP-A-208961 sind Aminoalkoholderivate der Formel (I) in der R¹ Wasserstoff oder Methyl, R³ Wasserstoff, R² Methyl oder Isopropyl und in der -(CH₂)ₙ-R⁴ eine unverzweigte Alkylkette mit 14 Kohlenstoffatomen bedeutet, beschrieben. In J. Med. Chem. 35, 2939-51 (1995) sind Aminoalkoholderivate der Formel (I) mit R¹, R³ Wasserstoff, R² Methyl oder Isobutyl und in der -(CH₂)ₙ-R⁴ eine unverzweigte Alkylkette mit 14 Kohlenstoffatomen bedeutet, beschrieben. In Biochem. J. 288, 167-73 (1992) ist eine Verbindung der Formel (I), mit R¹, R³ Wasserstoff, R² Isobutyl und in der -(CH₂)ₙ-R⁴ eine unverzweigte Alkylkette mit 10 Kohlenstoffatomen bedeutet, beschrieben. In J. Lipid Res. 13 (1), 86-91 (1972) und in DE-A-3418525 sind Verbindungen der Formel (I), in der R¹, R³ Wasserstoff, R² Ethyl und in der -(CH₂)ₙ-R⁴ eine unverzweigte Alkylkette mit 8, 10, 12, 14 und 16 Kohlenstoffatomen bedeutet, beschrieben. Alle Verbindungen sind als Zwischenprodukte ohne Angaben einer möglichen Verwendung als Arzneimittel beschrieben. Verbindungen der Formel (I), in der R¹, R³ Wasserstoff, R² Methyl oder Isobutyl und in der -(CH₂)ₙ-R⁴ (all-*cis*-R¹, R³ Wasserstoff, R² Methyl oder Isobutyl und in der -(CH₂)ₙ-R⁴ (all-*cis*-4,7,10,13)-Octadecatetraen ist, sind als Cannabinoid-rezeptorliganden in Life Sci. 56 (23/24), 2041-8 (1995) beschrieben worden.

In Lipids, Bd. 7, Nr. 1, 1972, S. 56-59 sind N-Alkanoyl-2-amino-2-methyl propanole, die therapeutisch zur Behandlung der Krabbe-Krankheit eingesetzt werden können.

Gegenstand der Erfindung ist daher auch die Verwendung der Verbindungen der Formel I 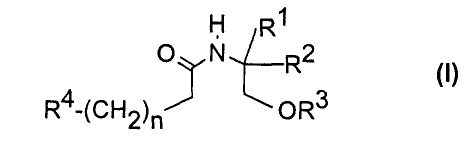
- R¹ =: Wasserstoff oder Methyl
- R² =: niederes geradkettiges oder verzweigtes Alkyl mit 1 bis 10 Kohlenstoffatomen
- R³ =: Wasserstoff oder niederes Alkyl
- n =: 0-12
- R⁴ =: Alkyl, Alkenyl oder Alkinyl mit 6 bis 24 Kohlenstoffatomen,
bedeuten,
sowie deren pharmakologisch unbedenkliche Salze und optischen Isomeren zur Herstellung von Arzneimitteln zur Behandlung von Knochenstoffwechselstörungen.

Niederes Alkyl soll in allen Fällen eine geradkettige oder verzweigte C₁-C₆-Alkylgruppe wie z.B. Methyl, Ethyl, Propyl, Isopropyl, Butyl, Isobutyl, Pentyl oder Hexyl, insbesondere Methyl, Ethyl, Propyl und Butyl darstellen.

Alkyl soll in allen Fällen eine geradkettige oder verzweigte C₆ - C₁₈-Alkyl-gruppe, wie z.B. Hexyl, Isohexyl, 2,2-Dimethylhexyl, 5-Methylhexyl, Heptyl, Isoheptyl, 6-Methylheptyl, Octyl, Isooctyl, Nonyl, Isononyl, Decyl, Isodecyl, Undecyl, Isoundecyl, Dodecyl, Isododecyl, Tridecyl, Isotridecyl, Tetradecyl, Isotetradecyl, Pentadecyl, Isopentadecyl, Hexadecyl, Heptadecyl, Isoheptadecyl oder Octadecyl, insbesondere Heptyl, Decyl und Dodecyl darstellen.

Alkenyl bedeutet in allen Fällen ein einfach oder mehrfach, gegebenfalls substituierter, ungesättigter Rest mit 6 - 20 Kohlenstoffatomen, wie z.B. Δ¹-Hexenyl, Δ¹-Octenyl, Δ⁹-Nonenyl, Δ¹-Decenyl, Δ¹⁰-Decenyl, Δ^{1,4}-Decadienyl, Δ ^{1,4,7}-Decatrienyl, Δ^{1,4,7,10}-Hexadeca-tetraenyl, Δ¹-Dodecenyl, Δ⁵-Dodecenyl, Δ^{1,4}-Undecadienyl, Δ¹⁴-Tetradecenyl, insbesondere Δ¹-Decenyl, Δ^{1,4}-Decadienyl, Δ ^{1,4,7}-Decatrienyl, wobei die Doppelbindungen cis oder trans, und bei mehrfach ungesättigten Verbindungen alle Kombinationen möglich sein können.

Alkinyl bedeutet in allen Fällen ein einfach oder mehrfach, gegebenenfalls substituierter, ungesättigter Rest mit 6 - 20 Kohlenstoffatomen, wie z.B. Δ¹-Decinyl, Δ¹-Noninyl, Δ^{1,3}-Tetradecadiinyl, Δ^{1,3}-Hexadecadiinyl, Δ^{1,3}-Octadecadiinyl, insbesondere Δ¹-Decinyl.

Verbindungen der allgemeinen Formel (I) enthalten mindestens ein asymmetrisches Kohlenstoffatom, daher sind auch optisch aktive Verbindungen der allgemeinen Formel (I) Gegenstand der vorliegenden Anmeldung.

Verbindungen der allgemeinen Formel (I) werden nach bekannten Verfahren zur Bildung von Carbonsäureamiden aus den Aminoalkoholen der allgemeinen Formel (II), 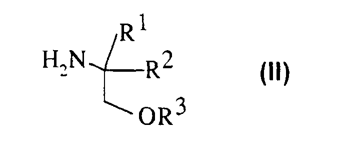 in der R¹, R² und R³ die oben angegebenen Bedeutungen haben,
und Carbonsäurederivaten der allgemeinen Formel (III), 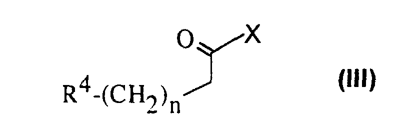 in der R⁴ und n die oben angegebenen Bedeutungen haben, und X eine Hydroxy- oder eine Aktivierungsgruppe sein kann, wobei falls X Hydroxy bedeutet, die Aktivierung der Carboxylgruppe nach dem Carbodiimidverfahren durchgeführt werden kann, und falls X eine Aktivierungsgruppe bedeutet, hierfür gemischte Anhydride, insbesondere mit Kohlensäureniederalkylestern wie Ethyl- oder Isobutylestern, oder aktive Ester, insbesondere p-Nitrophenyl-, 2.4.5-Trichlorphenyl-, N-Hydroxysuccinimid oder 1-Hydroxybenzotriazolester in Betracht kommen,
oder durch Kondensation mit Nitrilen der allgemeinen Formel (IV),

R⁴-(CH₂)ₙ₊₁-CN **(IV)**

in der R⁴ und n die oben angegebenen Bedeutungen haben, (vgl. Liebigs Ann. Chem. 986-96 (1979)),
erhalten.

Verbindungen der allgemeinen Formel (II) werden nach an sich bekannten Verfahren, vorzugsweise durch Reduktion von Aminosäuren, hergestellt oder sind käuflich.

Verbindungen der allgemeinen Formel (III) werden nach bekannten Verfahren aus Verbindungen der allgemeinen Formel (V)

R⁴-(CH₂)ₙ₊₁-COOH **(V)**

in der R⁴ und n die oben angegebenen Bedeutungen haben hergestellt.

Verbindungen der allgemeinen Formel (IV) werden nach bekannten Verfahren zur Synthese von Nitrilen hergestellt oder sind käuflich.
Verbindungen der allgemeinen Formel (V) werden nach bekannten Verfahren zur Kettenverlängerung bzw. Synthese von Carbonsäuren hergestellt oder sind käuflich.

Zu reinen Enantiomeren der Verbindungen der Formel (I) gelangt man durch den Einsatz von optisch aktiven Aminoalkoholen, die sich nach bekannten Verfahren, z.B. durch klassische Racematspaltung über Salzbildung mit optisch aktiven Säuren oder durch Reduktion optisch aktiver Aminosäuren herstellen lassen.

Verbindungen der Formel (I) können in flüssiger,fester oder in Form von Aerosolen oral, enteral, parenteral, topisch, nasal, pulmonal oder rectal in allen üblichen nichttoxischen pharmazeutisch akzeptierten Trägermaterialien, Adjuvantien und Zusätzen verabbreicht werden. Die Verbindungen der Formel (I) können auch lokal an/in den Knochen (evtl. unter chirurgischem Eingriff) appliziert werden. Der Begriff parenteral umfaßt dabei subcutane, intravenöse und intramuskuläre Zufuhr oder Infusionen. Orale Applikationsformen können z.B. Tabletten, Kapseln, Dragees, Sirupe, Lösungen, Suspensionen, Emulsionen, Elixiere etc. sein, die einen oder mehrere Zusätze aus den folgenden Gruppen enthalten können, wie z.B. Geschmacksstoffe, Süßstoffe, Farbstoffe und Konservierungsmittel. Orale Applikationsformen enthalten den wirksamen Bestandteil zusammen mit nichttoxischen, pharmazeutisch akzeptierten Trägermaterialien, die zur Herstellung von Tabletten, Kapseln, Dragees usw. geeignet sind, wie z.B. Calciumcarbonat, Natriumcarbonat, Lactose, Calciumphosphat oder Natriumphosphat; Stärke, Mannit, Methylcellulose, Talkum, hochdisperse Kieselsäuren, höhermolekulare Fettsäuren (wie Stearinsäure), Erdnußöl, Olivenöl, Paraffin, Miglyol, Gelatine, Agar-Agar, Magnesiumstearat, Bienenwachs, Cetylalkohol, Lecithin, Glycerol, tierische und pflanzliche Fette, feste hochmolekulare Polymere (wie Polyethylenglykole). Tabletten, Kapseln, Dragees usw. können mit einem entsprechenden Überzug, wie z.B. Glycerylmonostearat oder Glyceryldistearat versehen werden, so daß unerwünschte Nebenwirkungen im Magen verhindert werden, oder es durch die verzögerte Absorption im Gastrointestinaltrakt zu einer längeren Wirkungsdauer kommt. Als Injektionsmedium kommen vorzugsweise sterile injizierbare wäßrige oder ölige Lösungen oder Suspensionen zur Anwendung, welche die üblichen Zusätze, wie Stabilisierungsmittel und Lösungsvermittler enthalten. Derartige Zusätze können z.B. Wasser, isotonische Kochsalzlösung, 1,3-Butandiol, Fettsäuren (wie Ölsäure), Mono- und Diglyceride, oder Miglyol sein. Für die rectale Anwendung können alle geeigneten nicht irritierenden Zusätze verwendet werden, die bei normalen Temperaturen fest und bei Rectaltemperatur flüssig sind, wie z. B. Kakaobutter und Polyethylenglykol. Für die Anwendung als Aerosol kommen die pharmazeutisch üblichen Trägermedien zur Anwendung. Für den äußerlichen Gebrauch finden Cremes, Tinkturen, Gele, Lösungen oder Suspensionen usw. mit den pharmazeutisch üblichen Zusätzen Anwendung.

Die Applikation direkt an/in den Knochen (evtl. unter chirurgischem Eingriff) kann entweder in Lösung oder Suspension zweckmäßig durch Infusion oder Injektion (vorzugsweise lokal) oder trägergebunden erfolgen. Trägergebundene Verbindungen der Formel (I) können beispielsweise als Gele, Pasten oder als Beschichtung auf Implantaten appliziert werden.

Als Träger werden biokompatible und vorzugsweise bioabbaubare Materialien verwendet. Vorzugsweise induzieren die Materialien selbst zusätzlich noch die Wundheilung oder die Osteogenese.

Zur lokalen Applikation ist es bevorzugt, die Verbindungen der Formel (I) in polymere Gele oder Filme einzubetten, dadurch zu immobilisieren und diese Präparation direkt auf die zu behandelnde Stelle am Knochen aufzutragen. Derartige polymere Basisgele oder Filme bestehen beispielsweise aus Glycerin, Methylcellulose, Hyaluronsäure, Polyethylenoxiden und/oder Polyoxameren. Ebenfalls geeignet sind Kollagen, Gelatine und Alginate und sind beispielsweise in WO 93/00050 und WO 93/20859 beschrieben. Weitere Polymere sind Polymilchsäure (PLA) und Copolymere aus Milchsäure und Glykolsäure (PLPG) (Hollinger et al., J. Biomed. Mater. Res. 17 71-82 (1983)) sowie das Knochenderivat "Demineralized Bone Matrix" (DBM) (Guterman et al. Kollagen Rel. Res. 8 419-4319 (1988). Ebenfalls geeignet sind Polymere, wie sie zum Beispiel zur Adsorption für TGFß verwendet werden und in der EP-A-0 616 814 und der EP-A-0 567 391 beschrieben sind und synthetische Knochenmatrices gemäß WO 91/18558.

Ebenso geeignet als Träger für die Verbindungen der Formel (I) sind Materialien, die üblicherweise bei der Implantation von Knochenersatzstoffen oder von sonstigen therapeutischen Wirkstoffen verwendet werden. Solche Träger basieren beispielsweise auch auf Calciumsulfat, Tricalciumphosphat, Hydroxyapatit und Polyanhydriden. Außer diesen bioabbaubaren Trägern sind auch Träger geeignet, die nicht bioabbaubar sind, aber biokompatibel sind. Solche Träger sind beispielsweise gesinterter Hydroxylapatit, Bioglas, Aluminate oder andere keramische Materiaölien (z.B. Calcium-Aluminat-Phosphat). Diese Materialien werden bevorzugt in Kombination mit den bioabbaubaren Materialen, wie insbesondere Polymilchsäure, Hydoxylapatit, Kollagen oder Tricalciumphosphat angewendet. Weitere nicht abbaubare Polymere sind beispielsweise im US-Patent 4,164,560 beschrieben.

Besonders bevorzugt ist es, einen Träger zu verwenden, der die Verbindungen der Formel (I) kontinuierlich am Wirkort freisetzt. Hierfür besonders geeignet sind z.B. "slow release pellets" von Innovative Research of America, Toledo, Ohio, USA. Besonders bevorzugt werden Pellets verwendet, welche die Verbindungen der Formel (I) über mehrere Tage, vorzugsweise bis zu 100 Tagen bei einer täglichen Dosis von 1-10 mg/kg pro Tag, freisetzen.

Die Dosierung kann von verschiedenen Faktoren, wie Applikationsweise, Spezies, Alter und/oder individuellem Zustand abhängen. Die täglich zu verabreichende Dosis wirksamer Substanz liegt bei 0.01 mg bis ungefähr 100 mg/kg Körpergewicht, vorzugsweise bei 0.1 bis 10 mg/kg Körpergewicht und kann auf einmal oder mehrere Male verteilt appliziert werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Aminoalkohoiderivate:

### Bevorzugte Verbindungen (BV):

- (1): [1-(Hydroxymethyl)-ethyl]-octanamid
- (2): [1-(Hydroxymethyl)-propyl]-octanamid
- (3): [1-(Hydroxymethyl)-pentyl]-octanamid
- (4): [1-(Hydroxymethyl)-ethyl]-7-methyloctanamid
- (5): [1-(Hydroxymethyl)-butyl]-7-methyloctanamid
- (6): [1-(Hydroxymethyl)-propyl]-7,7-dimethyloctanamid
- (7): [1-(Hydroxymethyl)-pentyl]-7,7-dimethyloctanamid
- (8): [1-(Hydroxymethyl)-ethyl]-nonanamid
- (9): [1-(Hydroxymethyl)-butyl]-nonanamid
- (10): [1-(Hydroxymethyl)-ethyl]-4-methylnonanamid
- (11): [1-(Hydroxymethyl)-propyl]-8-methylnonanamid
- (12): [1-(Hydroxymethyl)-ethyl]-decanamid
- (13): [1-(Hydroxymethyl)-butyl]-decanamid
- (14): [1-(Hydroxymethyl)-propyl]-undecanamid
- (15): [1-(Hydroxymethyl)-pentyl]-undecanamid
- (16): [1 -(Hydroxymethyl)-ethyl]-10-methylundecanamid
- (17): [1 -(Hydroxymethyl)-butyl]-10-methylundecanamid
- (18): [1-(Hydroxymethyl)-propyl]-dodecanamid
- (19): [1-(Hydroxymethyl)-pentyl]-dodecanamid
- (20): [1 -(Hydroxymethyl)-pentyl]-11-methyldodecanamid
- (21): [1-(Hydroxymethyl)-butyl]-11-methyldodecanamid
- (22): [1-(Hydroxymethyl)-pentyll]-tridecanamid
- (23): [1-(Hydroxymethyl)-pentyl]-12-methyltridecanamid
- (24): [1-(Hydroxymethyl)-ethyl]-tetradecanamid
- (25): [1-(Hydroxymethyl)-pentyl]-tetradecanamid
- (26): [1 -(Hydroxymethyl)-butyl]-13-methyltetradecanamid
- (27): [1 -(Hydroxymethyl)-pentyl]-13-methyltetradecanamid
- (28): [1-(Hydroxymethyl)-ethyl]-pentadecanamid
- (29): [1-(Hydroxymethyl)-propyl]-pentadecanamid
- (30): [1-(Hydroxymethyl)-butyl]-pentadecanamid
- (31): [1 -(Hydroxymethyl)-butyl]-14-methylpentadecanamid
- (32): [1-(Hydroxymethyl)-pentyl]-14-methylpentadecanamid
- (33): [1-(Hydroxymethyl)-ethyl]-hexadecanamid
- (34): [1-(Hydroxymethyl)-ethyl]-15-methylhexadecanamid
- (35): [1 -(Hydroxymethyl)-propyl]-15-methylhexadecanamid
- (36): [1 -(Hydroxymethyl)-pentyl]-15-methylhexadecanamid
- (37): [1-(Hydroxymethyl)-ethyl]-heptadecanamid
- (38): [1-(Hydroxymethyl)-pentyl]-heptadecanamid
- (39): [1-(Hydroxymethyl)-proyl]-16 methylheptadecanamid
- (40): [1-(Hydroxymethyl)-pentyl]-16 methylheptadecanamid
- (41): [1-(Hydroxymethyl)-pentyl]-octadecanamid
- (42): [1 -(Hydroxymethyl)-butyl]-17-methyloctadecanamid
- (43): [1 -(Hydroxymethyl)-pentyl]-17-methyloctadecanamid
- (44): [1-(Hydroxymethyl)-ethyl]-nonadecanamid
- (45): [1-(Hydroxymethyl)-pentyl]-nonadecanamid
- (46): [1-(Hydroxymethyl)-ethyl]-18-methylnonadecanamid
- (47): [1-(Hydroxymethyl)-ethyl]-eicosanamid
- (48): [1-(Hydroxymethyl)-butyl]-eicosanamid
- (49): [1 -(Hydroxymethyl)-butyl]-19-methyleicosanamid
- (50): [1-(Hydroxymethyl)-ethyl]-heneicosanamid
- (51): [1-(Hydroxymethyl)-propyl]-docosanamid
- (52): [1-(Hydroxymethyl)-pentyl]-tricosanamid
- (53): [1-(Hydroxymethyl)-pentyl]-tetracosanamid
- (54): [1-(Hydroxymethyl)-butyl]-heptacosanamid
- (55): [1-(Hydroxymethyl)-pentyl]-heptacosanamid
- (56): [1-(Hydroxymethyl)-pentyl]-hexacosanamid
- (57): [1-(Hydroxymethyl)-ethyl]-heptacosanamid
- (58): [1-(Hydroxymethyl)-propyl]-octacosanamid
- (59): [1-(Hydroxymethyl)-butyl]-triacontanamid
- (60): [1-(Hydroxymethyl)-pentyl-6-heptenamid
- (61): [1-(Hydroxymethyl)-ethyl]-*trans*-9-hexadecenamid
- (62): [1-(Hydroxymethyl)-propyl]-*trans*-9-hexadecenamid
- (63): [1-(Hydroxymethyl)-pentyl]-*trans*-9-hexadecenamid
- (64): [1-(Hydroxymethyl)-propyl]-(all-cis-11,14,17)-eicosatrienamid
- (65): [1 -(Hydroxymethyl)-butyl]-(all-*cis*-11,14,17)-eicosatrienamid
- (66): [1-(Hydroxymethyl)-pentyl]-(all*-cis*-11,14,17)-eicosatrienamid
- (67): [1 -(Hydroxymethyl )-proyl]-cis-10-heptadecenamid
- (68): [1-(Hydroxymethyl)-pentyl]-*cis*-10-heptadecenamid
- (69): [1 -(Hydroxymethyl)-pentyl]-*cis*-10-nonadecenamid
- (70): [1-(Hydroxymethyl)-ethyl]-*cis*-3,*cis*-6-nonadienamid
- (71): [1-(Hydroxymethyl)-butyl]-cis-3,*cis*-6-nonadienamid
- (72): [1-(Hydroxymethyl)-ethyl]-*cis*-10-pentadecenamid
- (73): [1 -(Hydroxymethyl)-pentyl]*-cis*-10-pentadecenamid
- (74): [1-(Hydroxymethyl)-butyl]-*cis*-12-octadecenamid
- (75): [1-(Hydroxymethyl)*-*proyl]*-cis-*13-octadecenamid
- (76): [1-(Hydroxymethyl)-pentyl]-*cis*-13-octadecenamid
- (77): [1-(Hydroxymethyl)-ethyl]-*cis*-7-octadecenamid
- (78): [1-(Hydroxymethyl)-ethyl]-*cis*-8-eicosenamid
- (79): [1-(Hydroxymethyl)-butyl]-*cis*-8-eicosenamid
- (80): [1-(Hydroxymethyl)-ethyl]-*trans*-9-tetradecenamid
- (81): [1-(Hydroxymethyl)-propyl]-trans-9-tetradecenamid
- (82): [1-(Hydroxymethyl)-pentyl]-*trans*-9-tetradecenamid
- (83): [1 -(Hydroxymethyl)-pentyl]-cis-9,*cis*-11-octadecadienamid
- (84): [1-(Hydroxymethyl)-ethyl]*-cis-*9,*cis-*12-octadecadienamid
- (85): [1-(Hydroxymethyl)-butyl]-*cis*-9,*cis*-12-octadecadienamid
- (86): [1-(Hydroxymethyl)-propyl]-*trans*-9-octadecenamid
- (87): [1-(Hydroxymethyl)-butyl]-*trans*-9-octadecenamid
- (88): [1-(Hydroxymethyl)-ethyl]-*cis*-9-octadecenamid
- (89): [1-(Hydroxymethyl)-propyl]-*cis*-9-octadecenamid
- (90): [1 -(Hydroxymethyl)-ethyl]-(all-*trans*-9,11,13,15)-octadecatetraenamid
- (91): [1 -(Hydroxymethyl)-pentyl]-(all-trans-9,11,13,15)-octadecatetraenamid
- (92): [1-(Hydroxymethyl)-butyl]-(all-cis-9,11,13,15)-octadecatetraenamid
- (93): [1 -(Hydroxymethyl)-pentyl]-(all-*cis*-9,11,13,15)-octadecatetraenamid
- (94): [1-(Hydroxymethyl)-ethyl]-cis-11-octadecenamid
- (95): [1-(Hydroxymethyl)-pentyl]-*cis*-11-octadecenamid
- (96): [1-(Hydroxymethyl)-ethyl]-*cis*-13-docosenamid
- (97): [1-(Hydroxymethyl)-propyl]-(all-*cis*-13,16,19)-docosatrienamid
- (98): [1-(Hydroxymethyl)-pentyl]-(all-*cis*-13,16,19)-docosatrienamid
- (99): [1 -(Hydroxymethyl)-ethyl]-(all-cis-9,12,15)-octadecatrienamid
- (100): [1 -(Hydroxymethyl)-ethyl]-(all-*cis*-8,11,14)-eicosatrienamid
- (101): [1-(Hydroxymethyl)-propyl]-(all-*cis*-8,11,14)-eicosatrienamid
- (102): [1-(Hydroxymethyl)-butyl]-(all-*cis*-8,11,14)-eicosatrienamid
- (103): [1-(Hydroxymethyl)-pentyl]-(all-*cis*-8,11,14)-eicosatrienamid
- (104): [1-(Hydroxymethyl)-ethyl]-*trans*-11-octadecenamid
- (105): [1-(Hydroxymethyl)-pentyl]-*trans-*13-docosenamid
- (106): [1-(Hydroxymethyl)-propyl]-*trans*-9,*trans*-12-octadecadienamid
- (107): [1-(Hydroxymethyl)-ethyl]-cis-9-tetradecenamid
- (108): [1 -(Hydroxymethyl)-propyl]-*cis*-9-tetradecenamid
- (109): [1-(Hydroxymethyl)-butyl]-*cis*-9-tetradecenamid
- (110): [1-(Hydroxymethyl)-ethyl]-*cis*-9-hexadecenamid
- (111): [1-(Hydroxymethyl)-methylbutyl]-*cis*-9-hexadecenamid
- (112): [1-(Hydroxymethyl)-butyl]-*cis*-9-hexadecenamid
- (113): [1-(Hydroxymethyl)-ethyl]-10-undecenamid
- (114): [1-(Hydroxymethyl)-pentyl]-(all-*cis*,8,11,14)-eicosatrienamid
- (115): [1-(Hydroxymethyl )-pentyl](*-cis*-11, *cis*-14-eicosadienamid
- (116): [1-(Hydroxymethyl)-ethyl]-*cis*-11-eicosenamid
- (117): [1-(Hydroxymethyl)-pentyl-*cis*-11-eicosenamid
- (118): [1-(Hydroxymethyl)-ethyl]-*cis*-15-tetracosenamid
- (119): [1-(Hydroxymethyl)-propyl]-*cis*-15-tetracosenamid
- (120): [1 -(Hydroxymethyl)-pentyl]-11-dodecenamid
- (121): [1-(Hydroxymethyl)-ethyl]-9-decenamid
- (122): [1 -(Hydroxymethyl)-butyl]-16-heptadecenamid
- (123): [1-(Hydroxymethyl)-ethyl]-(all-*cis*-11,14,17)-eicosatrienamid
- (124): [1 -(Hydroxymethyl)-butyl]-(all-*cis*-11,14,17)-eicosatrienamid
- (125): [1 -(Hydroxymethyl)-pentyl]-(all-*cis*-11,14,17)-eicosatrienamid
- (126): [1-(Hydroxymethyl)-methylbutyl]-*cis*-13-eicosenamid
- (127): [1-(Hydroxymethyl)-ethyl]-*cis*-13,*cis*-13-docosadienamid
- (128): [1-(Hydroxymethyl)-propyl]-(all-*cis*-7,10,13,16)-docosatetraenamid
- (129): [1-(Hydroxymethyl)-ethyl]-22-tricosenamid
- (130): [1-(Hydroxymethyl)-ethyl]-9-tetradecinamid
- (131): [1-(Hydroxymethyl)-butyl]-9-tetradecinamid
- (132): [1 -(Hydroxymethyl)-ethyl]-13-eicosinamid
- (133): [1-(Hydroxymethyl)-ethyl]-10,12-nonacosadiinamid
- (134): [1-(Hydroxymethyl)-ethyl]-10,12-octadecadiinamid
- (135): [1 -(Hydroxymethyl)-pentyl]-10,12-octadecadiinamid
- (136): [1-(Hydroxymethyl)-butyl]-9-octadecinamid
- (137): [1-(Hydroxymethyl)-propyl]-9-octadecinamid
- (138): [1-(Hydroxymethyl)-methylbutyl]-9-octadecinamid
- (139): [1 -(Hydroxymethyl)-ethyl]-10-undecinamid
- (140): [1-(Hydroxymethyl)-butyl]-10,12-tricosadiinamid
- (141): [1-(Hydroxymethyl)-ethyl]-10,12-pentacosadiinamid
- (142): [1-(Hydroxymethyl)-ethyl]-10,12-pentacosadiinamind

Die nachfolgenden Beispiele zeigen einige der Verfahrensvarianten, die zur Synthese der erfindungsgemäßen Verbindungen verwendet werden können. Sie sollen jedoch keine Einschränkung des Erfindungsgegenstandes darstellen. Die Struktur der Verbindungen wurde durch ¹H- und gegebenfalls durch ¹³C-NMR-Spektroskopie gesichert. Die Reinheit der Substanzen wurde mittels C,H,N-Elementaranalyse, sowie dünnschichtchromatographisch bestimmt.

### Allgemeine Arbeitsvorschriften

### Allgemeine Arbeitsvorschrift A:

25 mmol Carbonsäure der Formel III werden in 125 ml THF gelöst. Nach Zugabe von 30 mmol 1,1'-Carbonyldiimidazol wird 10 min am Rückfluß gekocht. Bei Raumtemperatur werden 50 mmol Aminoalkohol der Formel II zugeben. Nach weiteren 3 Stunden am Rückfluß wird im Vakuum eingeengt. Der Rückstand wird in Diethylether aufgenommen, mit Wasser, 0.5 N NaOH, Wasser gewaschen. Die organische Phase wird über Magesiumsulfat getrocknet und im Vakuum eingeengt.

### Allgemeine Arbeitsvorschrift B:

Zu einer Lösung aus 25 mmol Carbonsäure der Formel III und 25 mmol Triethylamin in 100 ml abs. Dichlormethan tropft man bei -10°C eine Lösung aus 25 mmol Chlorameisensäureisobutylester gelöst in 25 ml abs. Dichlormethan zu. Nach 15 min wird eine Lösung aus 30 mmol Aminoalkohol der Formel II und 30 mmol Triethylamin in 75 ml abs. Dichlormethan zugetropft. Es wird noch 30 min bei -10°C nachgerührt bevor man langsam auf Raumtemperatur erwärmt. Das Reaktionsgemisch wird im Vakuum eingeengt, in Diethylether aufgenommen und mit Wasser, 0.5 N NaOH, Wasser gewaschen. Die organische Phase wird über Magnesiumsulfat getrocknet und im Vakuum eingeengt.

### Allgemeine Arbeitsvorschrift C:

Zu einer Lösung aus 25 mmol Aminoalkohol der Formel II und 25 mmol Triethylamin in 100 ml abs. Dichlormethan wird bei 10°C eine Lösung aus 25 mmol Carbonsäurechlorid der Formel III in 30 ml abs. Dichlormethan zugetropft. Nach 48 Stunden Rühren bei Raumtemperatur wird im Vakuum eingeengt, der Rückstand in Diethylether aufgenommen und mit Wasser, 0.5 N HCl und gesättigter NaCl gewaschen. Die organische Phase wird über Magnesiumsulfat getrocknet und im Vakuum eingeengt.

### Beispiel 1

### R-N-[1-(Hydroxymethyl)-propyl]-eicosanamid

- Carbonsäure:: Eicosansäure
- Alkohol:: *R*-2-Amino-1-butanol

Die Darstellung erfolgt nach der allg. Arbeitsvorschrift A. Ausbeute 84 % farblose Kristalle; Fp. 90-92°C

### Beispiel 2

### R-N-[1-(Hydroxymethyl)-propyl]-(all-cis-9,12,15)-octadecatrienamid

- Carbonsäure:: Linolensäure
- Alkohol:: *R*-2-Amino-1-butanol

Die Darstellung erfolgt nach der allg. Arbeitsvorschrift B. Die Aufreinigung erfolgte durch Chromatographie an Kieselgel mit Essigsäureethylester/Isohexan (1:1). Ausbeute 61 % farbloses Oel.

### Beispiel 3

### N-[1-(Hydroxymethyl)-pentyl]-eicosanamid

- Carbonsäure:: Eicosansäure
- Alkohol:: 2-Aminol-hexanol

Die Darstellung erfolgt nach der allg. Arbeitsvorschrift C: Ausbeute 65 % farblose Kristalle.

### Beispiel 4

### N-[1-(Hydroxymethyl)-pentyl]-cis-3,cis-6-nonadienamid

- Carbonsäure:: cis-3,*cis*-6-Nonadiensäure
- Alkohol:: 2-Amino1-hexanol

Die Darstellung erfolgt nach der allg. Arbeitsvorschrift B. Die Aufreinigung erfolgte durch Chromatographie an Kieselgel mit Essigsäureethylester/Isohexan (1:1). Ausbeute 35 % farbloses Oel.

### Beispiel 5

### N-[1-(Hydroxymethyl)-pentyl]-nonanamid

- Carbonsäure:: Nonansäure
- Alkohol:: 2-Amino1-hexanol

Die Darstellung erfolgt nach der allg. Arbeitsvorschrift A. Ausbeute 95 % farbloses Oel.

### Beispiel 6

### R-N-[1-(Hydroxymethyl)-3-methylbutyl]-(all-cis-9,12,15)-octadecatrienamid

- Carbonsäure:: Linolensäure
- Alkohol:: *R*-2-Amino-4-methyl-1-pentanol

Die Darstellung erfolgt nach der allg. Arbeitsvorschrift B. Die Aufreinigung erfolgte durch Chromatographie an Kieselgel mit Essigsäureethylester/Isohexan (1:1). Ausbeute 94 % farbloses Oel.

### Beispiel 7

### R-N-[1-Hydroxymethyl)-butyl]-(all-cis-9,12,15)-octadecatrienamid

- Carbonsäure:: Linolensäure
- Alkohol:: *R*-2-Amino-1-pentanol

Die Darstellung erfolgt nach der allg. Arbeitsvorschrift B. Die Aufreinigung erfolgte durch Chromatographie an Kieselgel mit Essigsäureethylester/Isohexan (1:1). Ausbeute 86 % farbloses Oel.

### Beispiel 8

### R-N-[1-Hydroxymethyl)-propy]-cis-3,cis-6-nonadienamid

- Carbonsäure:: *cis*-3,*cis*-6-Nonadiensäure
- Alkohol:: *R*-2-Amino-1-butanol

### Synthese von cis-3, cis-6-Nonadiensäure

*cis*-3,*cis*-6-Nonadien-1-ol (150 mmol) werden in 140 ml Aceton gelöst und mit einer Mischung aus 14 g Chromtrioxid in 42 ml Wasser und 12.6 ml conc Schwefelsäure versetzt. Nach 6 h bei Raumtemperatur wird mit 70 ml Wasser verdünnt und die Mischung mit Diethylether extrahiert. Die vereinigt. org. Phasen werden mit Sodalösung extrahiert, die Sodalösung wird angesäuert und mit Dichlormethan extrahiert. Nach dem Trocknen über Magnesiumsulfat wird eingeengt und man erhält ein farbloses Oel. (Ausbeute: 54 %)

Die Darstellung erfolgt nach der allg. Arbeitsvorschrift A. Ausbeute 95 % farbloses Oel.

### Beispiel 9

### R-N-[1-Hydroxymethyl)-pentyl]-(all-cis-9,12,15)-octadecatrienamid

- Carbonsäure:: Linolensäure
- Alkohol:: *R*-2-Amino1-hexanol

Die Darstellung erfolgt nach der allg. Arbeitsvorschrift B. Die Aufreinigung erfolgte durch Chromatographie an Kieselgel mit Essigsäureethylester/Isohexan (1:1). Ausbeute 48 % farbloses Oel.

### Beispiel 10

### R-N-[1-Hydroxymethyl)-pentyl]-cis-9,cis-12-octadecadienamid

- Carbonsäure:: Linolsäure
- Alkohol:: *R*-2-Amino1-hexano

Die Darstellung erfolgt nach der allg. Arbeitsvorschrift C. Die Aufreinigung erfolgte durch Chromatographie an Kieselgel mit Essigsäureethylester/Heptan (2:1). Ausbeute 55 % farbloses Oel.

### Beispiel 11

### N-[1-Hydroxymethyl)-pentyl]-cis-9-hexadecenamid

- Carbonsäure:: Palmitoleinsäure
- Alkohol:: 2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (1:1). Ausbeute 75 % farbloses Oel.

### Beispiel 12

### S-N-[1-Hydroxymethyl)-pentyl]-cis-9-octadecenamid

- Carbonsäure:: Oelsäure
- Alkohol:: S-2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 40 % farbloses amorphes Pulver.

### Beispiel 13

### S-N-[1 -Hydroxymethyl)-pentyl]-9-octadecinamid

- Carbonsäure:: Octadecinsäure
- Alkohol:: S-2-Amino-1 -hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 51 % farblose Kristalle.

### Beispiel 14

### R-N-[1-Hydroxymethyl)-pentyl]-cis-9-octadecenamid

- Carbonsäure:: Olesäure
- Alkohol:: *R*-2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 65 % farblose Kristalle.

### Beispiel 15

### S-N-[1-Hydroxymethyl)-pentyl]-trans-9-octadecenamid

- Carbonsäure:: Elaidinsäure
- Alkohol:: S-2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 55 % farblose Kristalle.

### Beispiel 16

### R-N-[1-Hydroxymethyl)-pentyl]-9-octadecinamid

- Carbonsäure:: Octadecinsäure
- Alkohol:: *R*-2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 61 % farblose Kristalle.

### Beispiel 17

### R-N-[1-Hydroxymethyl)-pentyl]-trans-9-octadecenamid

- Carbonsäure:: Elaidinsäure
- Alkohol:: *R*-2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 54 % farbloses amorphes Pulver.

### Beispiel 18

### R-N-[1-Hydroxymethyl)-pentyl]-eicosanamid

- Carbonsäure:: Eicosansäure
- Alkohol:: *R*-2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 49 % farblose Kristalle.

### Beispiel 19

### N-[1-Hydroxymethyl)-pentyl]-cis-9-tetradecenamid

- Carbonsäure:: Myristeoleinsäure
- Alkohol:: 2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 84 % farbloses Oel.

### Beispiel 20

### N-[1-Hydroxymethyl)-pentyl]-9-octadecinamid

- Carbonsäure:: Octadecinsäure
- Alkohol:: 2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (1:1). Ausbeute 65 % farbloses Oel.

### Beispiel 21

### N-[1-Hydroxymethyl)-pentyl]-trans-9-octadecenamid

- Carbonsäure:: Elaidinsäure
- Alkohol:: 2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (1:1). Ausbeute 80 % farbloses Oel.

### Beispiel 22

### N-[1-Hydroxymethyl)-pentyl]-9-tetradecinamid

- Carbonsäure:: Tetradecinsäure
- Alkohol:: 2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 92 % farbloses Oel.

### Beispiel 23

### S-N-[1-Hydroxymethyl)-pentyl]-cis-9,cis-12-octadecadienamid

- Carbonsäure:: Linolsäure
- Alkohol:: S-2-Amino-1-hexanol

Die Darstellung erfolgt nach der allg. Arbeitsvorschrift C. Die Aufreinigung erfolgte durch Chromatographie an Kieselgel mit Essigsäureethylester/Heptan (2:1). Ausbeute 47 % farbloses Oel.

### Beispiel 24

### N-[1-Hydroxymethyl)-pentyl]-cis-9,cis-12-octadecadienamid

- Carbonsäure:: Linolsäure
- Alkohol:: 2-Amino-1-hexanol

Die Darstellung erfolgte nach der allg. Arbeitsvorschrift B. Ausbeute 98 % farbloses Oel.

### Beispiel 25

### N-[1-Hydroxymethyl)-pentyl]-6-heptenamid

- Carbonsäure:: 6-Heptensäure
- Alkohol:: 2-Amino-1-hexanol

Die Darstellung erfolgte nach der allg. Arbeitsvorschrift B. Ausbeute 92 % farbloses Oel.

### Beispiel 26

### N-[1-Hydroxymethyl)-pentyl]-cis-9-octadecenamid

- Carbonsäure:: Oelsäure
- Alkohol:: 2-Amino-1-hexanol

Die Darstellung erfolgte nach der allg. Arbeitsvorschrift A. Ausbeute 78 % farbloses Oel.

### Beispiel 27

### R-N-[1-Hydroxymethyl)-propyl]-cis-9,cis-12-octadecadienamid

- Carbonsäure:: Linolsäure
- Alkohol:: *R*-2-Amino-1-butanol

Die Darstellung erfolgte nach der allg. Arbeitsvorschrift B. Die Aufreinigung erfolgte durch Chromatographie an Kieselgel mit Essigsäureethylester. Ausbeute 54 % farbloses Oel.

### Beispiel 28

### S-N-[1-Hydroxymethyl)-propyl]-cis-9,cis-12-octadecadienamid

- Carbonsäure:: Linolsäure
- Alkohol:: S-2-Amino-1-butanol

Die Darstellung erfolgte nach der allg. Arbeitsvorschrift B. Die Aufreinigung erfolgte durch Chromatographie an Kieselgel mit Essigsäureethylester. Ausbeute 48 % farbloses Oel.

### Beispiel 29

### N-[1-Hydroxymethyl)-ethyl]-trans-9-octadecenamid

- Carbonsäure:: Elaidinsäure
- Alkohol:: 2-Amino-1-propanol

Die Darstellung erfolgte nach der allg. Arbeitsvorschrift B. Die Aufreinigung erfolgte durch Chromatographie an Kieselgel mit Essigsäureethylester. Ausbeute 72 % farbloses Oel.

### Beispiel 30

### N-[1-Hydroxymethyl)-butyl]-cis-9-octadecenamid

- Carbonsäure:: Oelsäure
- Alkohol:: 2-Amino-1-pentanol

Die Darstellung erfolgt nach der allg. Arbeitsvorschrift C. Die Aufreinigung erfolgte durch Chromatographie an Kieselgel mit Essigsäureethylester/Heptan (3:1). Ausbeute 88 % farbloses Oel.

### Beispiel 31

### N-[1-Hydroxymethyl)-ethyl]-9-octadecinamid

- Carbonsäure:: Octadecinsäure
- Alkohol:: 2-Amino-1-propanol

Die Darstellung erfolgt nach der allg. Arbeitsvorschrift C. Die Aufreinigung erfolgte durch Chromatographie an Kieselgel mit Essigsäureethylester/Heptan (1:1). Ausbeute 68 % farbloses Oel.

### Beispiel 32

Verbindungen der Formel (I) wurden in einem DNA-Synthese-Assay in Primärkulturen von Osteoblasten aus fetalen Rattenkalvarien untersucht. Die Experimente wurden in Anlehnung an Pfeilschifter et al., Endocrinology 126, 703 (1990) durchgeführt.

Die Gewinnung von primären Osteoblasten erfolgte durch sequentielle Abdauung mittels Collagenase aus fetalen Rattenkalvarien. Dabei wurden 5 Zellfraktionen erhalten. Der Pool aus den Zellfraktionen 3-5 wurde *in vitro* kultiviert. Die Kultur der Zellen erfolgte in einem Inkubator bei einer relativen Luftfeuchte von 95%, einem CO₂-Gehalt von 5% und einer Temperatur von 37°C. Die Untersuchungen der Prüfsubstanzen erfolgte in Kulturen der ersten, zweiten oder dritten Zellpassage. Für die Untersuchungen wurden die Zellen mindestens 76 Stunden vor Aufgabe der Prüfsubstanzen in einer Zellzahl von 7×10³ Zellen (in 100 µl Kulturmedium) / Well in Mikrotiterplatten ausgesät.

Dabei fand als Kulturmedium MEM Dulbecco (plus 4,5 g/l Glukose plus 3,7g/l NaHCO₃ ohne Glutamin) Verwendung, dem 5% fetales Kälberserum (FKS) und 5000 U/ml Penicillin/Streptomycin zugesetzt waren. Unmittelbar vor Zugabe der Prüfsubstanzen zur Zellkultur erfolgte ein Austausch des Mediums gegen 150 µl Medium, das anstelle von FKS 1 mg/ml Rinderserumalbumin (RSA) enthielt. Prüfsubstanzen wurden in den gewünschten Konzentrationen dem RSA-haltigen Medium zugesetzt. Als Positivkontrolle wurde TGFß₁ (Transforming growth factor ß₁) in Konzentrationen von 0.1-0.2 ng/ml mitgeführt. Pro (Positiv-) Kontrolle bzw. Substanzkonzentration wurden Dreifachbestimmungen durchgeführt. Die Inkubation der Zellkulturen mit Prüfsubstanzen erfolgte 24 Stunden, in den letzten 3 Stunden zusätzlich unter Anwesenheit der Thymidinsonde (1µCi Methyl-³H-Thymidin in 20µl PBS-Lösung). Am Ende der Inkubationszeit wurden die Zellkulturen 3x mit 0.9%iger Kochsalzlösung gewaschen und anschließend mit je 100µl Flüssigszintillator (OptiPhase Supermix TM® ) versetzt. Im Anschluß daran erfolgte die Vermessung der in die DNA eingebauten Radioaktivität in einem Flüssigszintillationscounter (1450 MicroBeta® ) in cpm. Bei der Auswertung dienten Zellkulturen, die ausschließlich RSA-haltiges Medium erhalten hatten, als Kontrollen (100 %).

**Tabelle I:**

| DNA-Synthese-Rate primärer osteoblastärer Zellen aus fetalen Ratten-kalvarien in Prozent im Vergleich zur Kontrolle (=100%) | | |
|---|---|---|
| **Verbindung** | **BSP.** | **1 µg/ml** |
| N-[1-Hydroxymethyl)-pentyl]-(all-cis-9,12,15)-octadecatrienamid | 9 | 367 |
| N-[1-Hydroxymethyl)-pentyl]-cis-9,cis-12-octadecadienamid | 10 | 475 |
| N-[1-Hydroxymethyl)-pentyl]-cis-9-hexadecenamid | 11 | 191 |
| N-[1-Hydroxymethyl)-pentyl]-cis-9-tetradecenamid | 19 | 298 |
| N-[1-Hydroxymethyl)-pentyl]-9-octadecinamid | 20 | 250 |
| N-[1-Hydroxymethyl)-pentyl]-trans-9 octadecenamid | 21 | 188 |
| N-[1-Hydroxymethyl)-pentyl]-cis-9,cis-12-octadecadienamid | 24 | 223 |
| N-[1-Hydroxymethyl)-pentyl]-cis-9-octadecenamid | 26 | 296 |
| N-[1-Hydroxymethyl)-propyl]-cis-9,cis-12-octadecadienamid | 27 27 | 252 |

## Patentansprüche

1. Verwendung von Verbindungen der Formel I 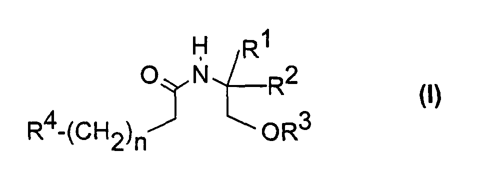 in der
R¹ = Wasserstoff oder Methyl
R² = niederes geradkettiges oder verzweigtes Alkyl mit 1 bis 10 Kohlenstoffatomen
R³ = Wasserstoff oder niederes Alkyl mit 1 bis 6 Kohlenstoffatomen
n = 0-12
R⁴ = Alkyl, Alkenyl oder Alkinyl mit 6 bis 24 Kohlenstoffatomen,
sowie deren pharmakologisch unbedenkliche Salze und optische Isomere zur Herstellung von Arzneimitteln zur Behandlung von Knochenstoffwechselstörungen.

2. Verbindungen der Formel Ia 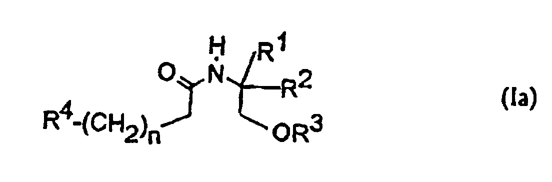 in der
R¹ = Wasserstoff oder Methyl, wobei für den Fall, daß R² = Methyl, R¹ = Wasserstoff ist,
R² = niederes geradkettiges oder verzweigtes Alkyl mit 1 bis 10 Kohlenstoffatomen
R³ = Wasserstoff oder niederes Alkyl mit 1 bis 6 Kohlenstoffatomen
n = 0-12
R⁴ = Alkyl, Alkenyl oder Alkinyl mit 6 bis 24 Kohlenstoffatomen,
wobei für den Fall, daß
R⁴ Alkyl bedeutet, -(CH₂)ₙ-R⁴ keine unverzweigte Alkylkette mit 8, 10, 12, 14 oder 16 Kohlenstoffatomen sein darf,
und für den Fall, daß
R² Methyl, Isopropyl oder Isobutyl bedeutet, -(CH₂)ₙ-R⁴ nicht (all-*cis*-4,7,10,13)- Octadecatetraen sein darf und keine unverzweigte Kette mit 6 Kohlenstoffatomen sein darf,
sowie deren pharmakologisch unbedenkliche Salze und optischen Isomeren.

3. Verfahren zur Herstellung von Verbindungen der Formel Ia 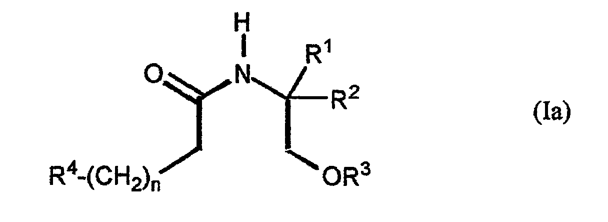 gemäß Anspruch 2
dadurch gekennzeichnet, daß man in an sich bekannter Weise eine Verbindung der Formel II 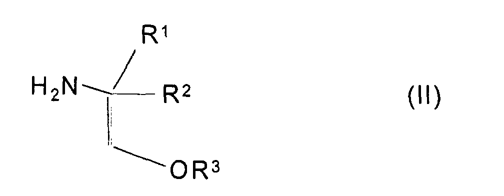 in der R^{1,} R² und R³ die angegebenen Bedeutungen haben,
entweder
(a) mit einer Verbindung der Formel III 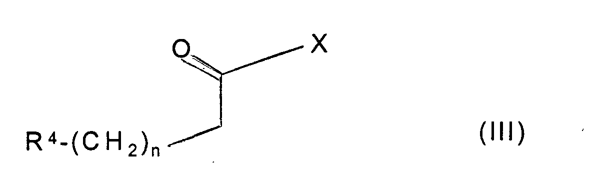 in der R⁴ und N die angegebenen Bedeutungen haben und X eine Aktivierungsgruppe darstellt,
umsetzt oder
(b) mit einer Verbindung der Formel IV
R⁴-(CH₂)ₙ₊₁-CN (IV)
in der R4 und n die angegebenen Bedeutungen haben,
umsetzt,
und anschließend gewünschtenfalls die erhaltenen Verbindungen in andere Verbindungen der Formel Ia in pharmakologisch verträgliche Salze oder in optische Isomere überführt.

4. Arzneimittel, enthaltend mindestens eine Verbindung der Formel Ia gemäß Anspruch 2 neben üblichen Träger- und Hilfsstoffen.

5. Verwendung von Verbindungen der Formel Ia gemäß Anspruch 2 zur Herstellung von Arzneimitteln zur Behandlung von Knochenstoffwechselstörungen.

## Claims

1. Use of compounds of formula I 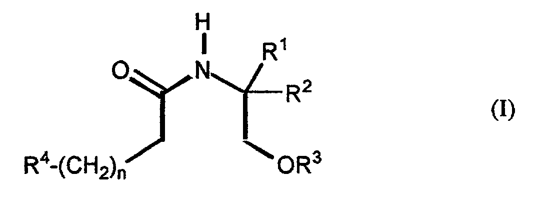 in which
R¹ = hydrogen or methyl
R² = lower straight-chained or branched alkyl with 1 to 10 carbon atoms
R³ = hydrogen or lower alkyl with 1 to 6 carbon atoms
n = 0-12
R⁴ = alkyl, alkenyl or alkynyl with 6 to 24 carbon atoms,
as well as their pharmacologically acceptable salts and optical isomers for the production of medicaments for the treatment of disorders of bone metabolism.

2. Compounds of formula Ia 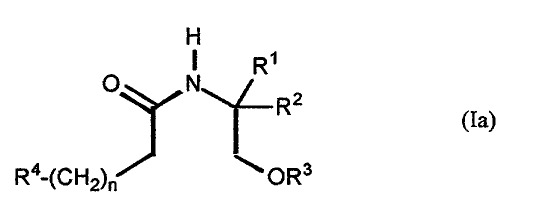 in which
R¹ = hydrogen or methyl, wherein R¹ = hydrogen in the case that R² = methyl,
R² = lower straight-chained or branched alkyl with 1 to 10 carbon atoms
R³ = hydrogen or lower alkyl with 1 to 6 carbon atoms
n = 0-12
R⁴ = alkyl, alkenyl or alkynyl with 6 to 24 carbon atoms,
wherein
-(CH₂)ₙ-R⁴ may not be an unbranched alkyl chain with 8, 10, 12, 14 or 16 carbon atoms in the case that R⁴ signifies alkyl
and
-(CH₂)ₙ-R⁴ may not be (all-*cis*-4,7,10,13)-octadecatetraene and may not be an unbranched chain with 6 carbon atoms in the case that R² signifies methyl, isopropyl or isobutyl,
as well as their pharmacologically acceptable salts and optical isomers.

3. A process for the manufacture of compounds of formula Ia 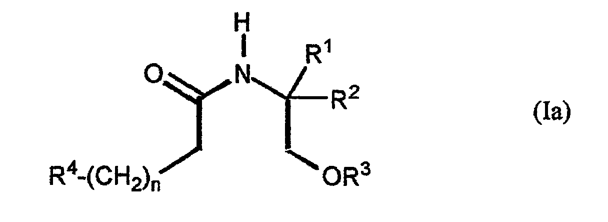 in accordance with claim 2,
characterized by reacting a compound of formula II 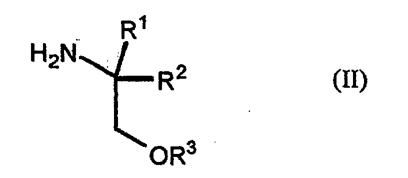 in which R¹, R² and R³ have the given significances,
in a manner known per se either
(a) with a compound of formula III 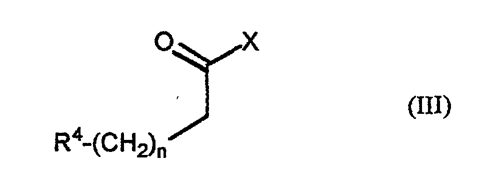 in which R⁴ and N have the given significances and X represents an activating group, or
(b) with a compound of formula IV
R⁴-(CH₂)ₙ₊₁-CN (IV)
in which R⁴ and n have the given significances, and subsequently, if desired, converting the compounds obtained into other compounds of formula Ia, into pharmacologically acceptable salts or into optical isomers.

4. Medicaments containing at least one compound of formula Ia in accordance with claim 2 in addition to conventional carriers and adjuvants.

5. The use of compounds of formula Ia in accordance with claim 2 for the production of medicaments for the treatment of disorders of bone metabolism.

## Revendications

1. Utilisation de composés de formule I : 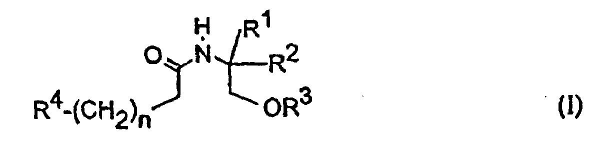 dans laquelle
**R**^{**1**} est un atome d'hydrogène ou le groupe méthyle,
**R**^{**2**} est un groupe alkyle inférieur à chaîne droite ou ramifiée ayant de 1 à 10 atomes de carbone,
**R**^{**3**} est un atome d'hydrogène ou un groupe alkyle inférieur ayant de 1 à 6 atomes de carbone,
n vaut de 0 à 12,
**R**^{**4**} est un groupe alkyle, alcényle ou alcynyle ayant de 6 à 24 atomes de carbone,
ainsi que leurs sels inoffensifs du point de vue pharmacologique et leurs isomères optiques, pour préparer des médicaments destinés au traitement des troubles du métabolisme osseux.

2. Composés de formule la : 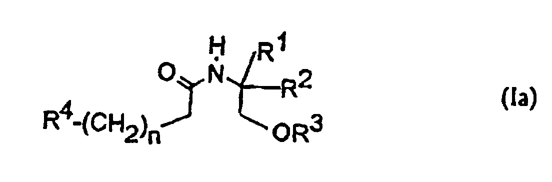 dans laquelle
R¹ est un atome d'hydrogène ou le groupe méthyle, où, quand R² est le groupe méthyle, R¹ est un atome d'hydrogène,
R² est un groupe alkyle inférieur à chaîne droite ou ramifiée ayant de 1 à 10 atomes de carbone,
R³ est un atome d'hydrogène ou un groupe alkyle inférieur ayant de 1 à 6 atomes de carbone,
n vaut de 0 à 12,
R⁴ xest un groupe alkyle, alcényle ou alcynyle ayant de 6 à 24 atomes de carbone,
où, quand R⁴ est un groupe alkyle, -(CH₂)ₙ-R⁴ ne peut représenter une chaîne alkyle non-ramifiée ayant 8, 10, 12, 14 ou 16 atomes de carbone,
et, quand R² est le groupe méthyle, isopropyle ou isobutyle, -(CH₂)ₙ-R⁴ ne peut être le groupe (tout-*cis*-4,7,10,13)-octadécatétraène et ne peut être une chaîne non-ramifiée ayant 6 atomes de carbone,
et leurs sels inoffensifs du point de vue pharmacologique et isomères optiques.

3. Procédé de préparation de composés de formule Ia : 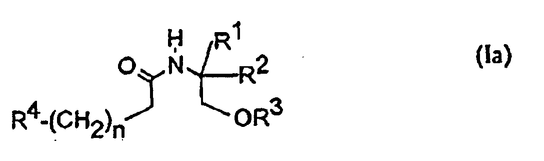 selon la revendication 2, caractérisé en ce qu'on fait réagir d'une manière connue en soi un composé de formule II : 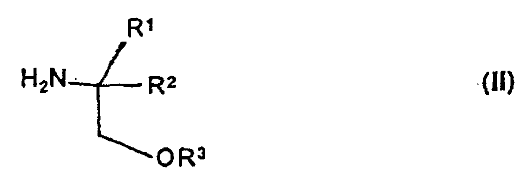 dans laquelle R¹, R² et R³ ont les significations indiquées,
(a) avec un composé de formule III: 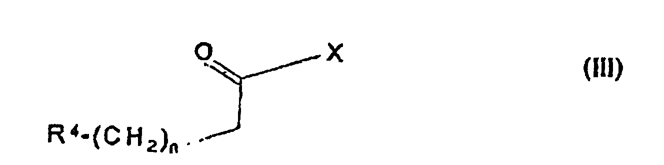 dans laquelle R⁴ et n ont les significations Indiquées et X est un groupe d'activation, ou bien
(b) avec un composé de formule IV :
R⁴-(CH₂)ₙ₊₁-CN (IV)
dans laquelle R⁴ et n ont les significations indiquées,
puis, si on le souhaite, on convertit les composés obtenus en d'autres composés de formule la, en des sels compatibles du point de vue pharmacologique ou en des isomères optiques.

4. Médicament contenant au moins un composé de formule 1a selon la revendication 2, en plus d'excipients et adjuvants usuels.

5. Utilisation de composés de formule la selon la revendication 2 pour préparer des médicaments destinés au traitement des troubles du métabolisme osseux.
